# EUROPÄISCHE PATENTSCHRIFT

(11) **EP 3 192 333 B1**
(45) Veröffentlichungstag und Bekanntmachung des Hinweises auf die Patenterteilung: **05.01.2022**
(21) Anmeldenummer: 15794058.6
(22) Anmeldetag: 25.08.2015
(51) Int. Cl.: H05H 1/24, H01J 37/32, A61L 2/14, A61N 5/06, A61B 18/00, A61B 18/04, A61B 18/14

(54) **ELEKTRODENANORDNUNG ZUR AUSBILDUNG EINER DIELEKTRISCH BEHINDERTEN PLASMAENTLADUNG**
ELECTRODE ARRANGEMENT FOR FORMING A DIELECTRIC BARRIER PLASMA DISCHARGE
ENSEMBLE D'ÉLECTRODES POUR FORMER UNE DÉCHARGE DE PLASMA À BARRIÈRE DIÉLECTRIQUE

(30) Priorität: 11.09.2014 DE 102014013716
(43) Veröffentlichungstag der Anmeldung: 19.07.2017
(73) Patentinhaber: Cinogy GmbH, 37115 Duderstadt (DE)
(72) Erfinder: TRUTWIG, Leonhard, 37115 Duderstadt (DE); HAHNL, Mirko, 37339 Berlingerode (DE); STORCK, Karl-Otto, 37115 Duderstadt (DE); KOPP, Matthias, 37434 Gieboldehausen (DE); SCHÄFER, Annika, 37081 Göttingen (DE); WANDKE, Dirk, 37308 Heilbad Heiligenstadt (DE)
(74) Vertreter: Gramm, Lins & Partner Patent- und Rechtsanwälte PartGmbB
(86) Internationale Anmeldenummer: PCT/DE2015/000422
(87) Internationale Veröffentlichungsnummer: WO 2016/037599

(56) Entgegenhaltungen:
- EP-A1- 2 170 022
- DE-A1- 10 203 543
- DE-B4-102009 060 627
- US-A1- 2013 345 620
- US-A1- 2014 182 879

## Beschreibung

Die Erfindung betrifft eine Elektrodenanordnung zur Ausbildung einer dielektrisch behinderten Plasmaentladung zwischen einer flächigen Oberfläche der Elektrodenanordnung und einer als Gegenelektrode dienenden, zu behandelnden Oberfläche, an der sich ein Fluid ansammeln kann, mit einer flächigen zusammenhängenden Elektrode, die mittels eines Anschlusses mit einer Hochspannungsquelle verbindbar ist und die in einem flächigen Dielektrikum mit Ausnahme des Anschlusses für die Hochspannungsquelle vollständig eingebettet ist, wobei das Dielektrikum eine Oberseite und eine die zur behandelnden Oberfläche zeigende flächige Ober-fläche bildende Unterseite ausbildet.

Eine derartige flächige Elektrodenanordnung, die flexibel ausgebildet sein kann, ist durch DE 10 2009 060 627 B4 bekannt. Die flächige Elektrode ist dabei eingebettet zwischen einer Dielektrikumunterseite und einer Dielektrikumoberseite, die sich jeweils in der Fläche über die Elektrode hinaus erstrecken und so auch den schmalen Rand der Elektrode abdecken, sodass eine Berührung der die Hochspannung führenden Elektrode ausgeschlossen ist. Ausgeschlossen ist ferner eine solche Annäherung an die Elektrode, dass ein Funke überspringen könnte. Vielmehr verhindert das Dielektrikum einen Stromfluss von der Elektrode zu der zu behandelnden Oberfläche, die als Gegenelektrode dient. Die Elektrodenanordnung weist somit keine eigene Gegenelektrode auf. Um die Ausbildung eines Plasmas in der Luftschicht zwischen der zu behandelnden Oberfläche und dem Dielektrikum sicherzustellen, kann bei einer glatten zu behandelnden Oberfläche die Unterseite der Elektrodenanordnung, die zu der zu behandelnden Fläche zeigt, mit vorstehenden Noppen ausgebildet sein, die mit ihrer Oberseite auf der zu behandelnden Oberfläche aufliegen und durchgehende Zwischenräume aufweisen, in denen sich das Plasma ausbilden kann, wenn an die Hochspannungselektrode eine Hochspannung, insbesondere eine Wechselhochspannung angelegt wird.

Eine derartige flächige Elektrodenanordnung lässt sich auf die zu behandelnde Oberfläche auflegen, wobei die zu behandelnde Oberfläche insbesondere auch die Haut eines menschlichen oder tierischen Körpers sein kann. Die Plasma-behandlung führt dabei zu einer porentiefen Desinfektion der Haut und verbessert die Aufnahmefähigkeit der Haut für pflegende Stoffe, die auf die behandelte Haut aufgebracht werden. '

Des Weiteren ist aus US 2013/0345620 A1 eine Elektrodenanordnung zur Ausbildung einer Plasmaentladung bekannt, die der Behandlung einer Hautoberfläche dient. Die Elektrodenanordnung weist eine flächige Elektrode auf, welche mit einer Hochspannungsquelle verbindbar ist, und eine Dielektrikumsschicht, welche die Elektrode im Wesentlichen umgibt.

Es ist auch bekannt, dass eine Plasmabehandlung für eine Wundheilung vorteilhaft sein kann. Gemäß DE 10 2009 047 220 A1 wird in einem stiftähnlichen Gerät, das von einem Behandlungsgas durchströmt wird, ein Plasma erzeugt, das an einer düsenähnlich ausgeformten Stirnseite des Geräts austritt und auf die zu behandelnde Haut bzw. Wunde geleitet werden kann.

Ein nach dem gleichen Prinzip arbeitendes ähnliches Gerät ist in EP 2 160 081 A1 offenbart.

DE 10 2011 001 416 A1 offenbart eine flächige flexible Wundbehandlungseinrichtung, bei der zwei Flächenelektroden durch miteinander verwobene, isolierte elektrische Leiter gebildet sind. Zwischen den Leitern bildet sich die Hochspannung aus, die in den Luftzwischenräumen ein Plasma entstehen lassen soll. Hierzu ist erforderlich, dass die gesamte Elektrodenanordnung gasdurchlässig ist. Wird eine Isolierung der Elektrodendrähte schadhaft, kommt es zu Funkenüberschlägen zwischen den Hochspannungselektroden, die schwere Schädigungen auf der Hautfläche verursachen können, auf der die Elektrodenanordnung mit einer Wundkontaktschicht aufliegt. Die Elektrodenanordnung erlaubt eine gewisse Abführung einer Sekretflüssigkeit von der Wundfläche, ist aber sicherheitstechnisch nicht oder nur schwierig zu beherrschen.

Ferner offenbart US 2014/0182879 A1 eine Elektrodenanordnung mit den im Oberbegriff des Anspruchs 1 definierten Merkmalen.

Der vorliegenden Erfindung liegt die Aufgabe zugrunde, eine Elektrodenanordnung der eingangs erwähnten Art in ihrem Anwendungsbereich zu erweitern, ohne eine Beeinträchtigung der Sicherheit hinnehmen zu müssen und ohne komplizierte Strukturen zu realisieren.

Diese Aufgabe wird erfindungsgemäß bei einer Elektrodenanordnung der eingangs erwähnten Art dadurch gelöst, dass die flächige zusammenhängende Elektrode über ihre Fläche verteilte, zur Ableitung von Fluid von der zu behandelnden Oberfläche ausgebildete Durchgangsöffnungen aufweist und dass das Dielektrikum mit sich von der Unterseite bis zur Oberseite erstreckenden Durchgangsöffnungen versehen ist, die mit den Durchgangsöffnungen der Elektrode fluchten und kleinere Abmessungen als die Durchgangsöffnungen der Elektrode aufweisen, sodass das Dielektrikum auch im Bereich der Durchgangsöffnungen die Elektrode vollständig abdeckt.

Die erfindungsgemäße Elektrodenanordnung ist im Prinzip ähnlich aufgebaut wie die aus der DE 10 2009 060 627 B4 bekannte Elektrodenanordnung und kann alle damit verbundenen Vorteile ebenfalls realisieren. Die Elektrodenanordnung ist jedoch erfindungsgemäß so ausgebildet, dass sie zwar einerseits aus flächigen zusammenhängenden Teilen besteht, nämlich der flächigen zusammenhängenden Elektrode und dem flächigen zusammenhängenden Dielektrikum, dass jedoch dennoch durch die Durchgangsöffnungen des Dielektrikums ein Fluid aus dem Zwischenraum zwischen der Unterseite des Dielektrikums und der zu behandelnden Oberfläche abführbar ist, ohne dass hierdurch eine Sicherheitsbeeinträchtigung der Elektrode in Kauf zu nehmen wäre. Vielmehr bildet das Dielektrikum von der Unterseite zur Oberseite verlaufende kleine Kanäle aus, die allseitig durch eine ausreichend dicke Dielektrikumschicht begrenzt sind, sodass keine Gefahr eines unmittelbaren Kontakts mit der die Hochspannung führenden Elektrode besteht. Erfindungsgemäß ist somit der flächige Körper der Elektrodenanordnung selbst für die Ableitung von Fluid durch die Kanäle geeignet, sodass für die Ableitung von Fluid, insbesondere Wundsekret und etwaige entstehende Gase, keine besondere Anordnung vorgesehen werden muss.

Auch die erfindungsgemäße Elektrodenanordnung kann in einer bevorzugten Ausführungsform eine mit vorstehenden Noppen versehene Unterseite des Dielektrikums aufweisen, wobei die Noppen die Höhe eines Freiraums definieren, wenn die Noppen auf der zu behandelnden Fläche aufliegen. Der Freiraum dient zumindest teilweise zur Ausbildung eines Plasmas in einem Gas, beispielsweise Luft, das sich in dem Freiraum befindet. Die Durchgangsöffnungen befinden sich vorzugsweise zwischen den Noppen. Zumindest einige der Durchgangsöffnungen können sich aber auch durch die Noppen hindurch erstrecken.

Vorzugsweise besteht das Dielektrikum aus einem gießbaren Kunststoff. Dabei ist es möglich, dass das die Elektrode einbettende Dielektrikum einstückig durch Umspritzen der Elektrode hergestellt wird. Werkzeugtechnisch einfacher ist es allerdings, wenn das Dielektrikum zweiteilig hergestellt wird, nämlich aus einem Unterteil und einem Oberteil, wobei die Elektrode in das Unterteil oder Oberteil eingelegt wird und dann das Dielektrikum durch Auflegen des anderen Teils und Verbinden der beiden Teile miteinander geschlossen wird. Diese Verbindung kann dadurch entstehen, dass das zweite Teil auf das erste Teil, in das die Elektrode bereits eingelegt ist, aufgespritzt wird, sodass sich eine materialschlüssige Verbindung ergibt. Darüber hinaus ist es möglich, die beiden Teile des Dielektrikums durch Verschweißung oder Verklebung miteinander zu verbinden.

In einer bevorzugten Ausführungsform wird der von dem Dielektrikum freiliegenden Anschluss der Elektrode mit einer Kontaktanordnung verbunden, die den Anschluss klemmend und isolierend übergreift. Dabei kann die Kontaktanordnung vorzugsweise einen klemmenden Zustand und einen Ausgangszustand aufweisen, wobei im klemmenden Zustand ein Hochspannungskontakt mit einer Vorspannung gegen den Anschluss der Elektrode drückt und im Ausgangszustand der Hochspannungskontakt durch ein mit dem Übergang vom Ausgangszustand in den klemmenden Zustand bewegbares Isolierstück abgedeckt sein. Hierbei ist es besonders vorteilhaft, wenn die Kontaktanordnung ein Gehäuse mit einem einseitig offenen Schlitz für den Anschluss der Elektrode aufweist und wenn in dem Gehäuse eine Klemmanordnung zum Andrücken des Hochspannungskontakts an den Anschluss der Elektrode gelagert ist.

Durch das bewegbare Isolierstück wird der Hochspannungskontakt im Ausgangszustand abgedeckt, sodass eine versehentliche Kontaktierung eines Körperteils mit dem Hochspannungskontakt nicht möglich ist. Hierzu muss das bewegbare Isolierstück erst von dem Hochspannungskontakt weggeschoben werden.

Der Hochspannungskontakt ist vorzugsweise ein zylindrischer Kontakt, der in eine Ausnehmung des Dielektrikums eingreift, die bis zu einem flächigen Anschlussstück der Elektrode ragt. Das flächige Anschlussstück ist vorzugsweise zungenförmig ausgebildet und vom Dielektrikum, mit Ausnahme der Ausnehmung, umschlossen, sodass mit dem zungenförmigen Anschlussstück das bewegbare Isolierstück der Kontaktanordnung verschoben werden kann, um danach den Hochspannungskontakt in die Ausnehmung des Dielektrikums, und damit gegen die leitende Oberfläche des Anschlussstücks der Elektrode drücken zu können.

Die erfindungsgemäße Elektrodenanordnung eignet sich insbesondere als Wundauflage und ist daher vorzugsweise insgesamt flexibel ausgebildet, also mit einem flexiblen Dielektrikum und einer flexiblen Elektrode. Dabei ist es zweckmäßig, wenn die Unterseite des Dielektrikums mit einer als Wundauflage geeigneten Schicht bedeckt ist. Diese Schicht sollte die Ausbildung des Plasmas zwischen der Unterseite des Dielektrikums und der zu behandelnden Oberfläche, hier in Form einer Wunde, nicht behindern. Als Wundauflage kommen daher übliche Mull-Zellulose und sonstige Auflagen infrage. Besonders bevorzugt ist es, wenn auf die Oberfläche des Dielektrikums eine Schicht aus einer festen, offenporigen Matrix aus einem pflegenden oder heilungsfördernden Material angeordnet ist. Die Schicht kann unmittelbar auf das Dielektrikum aufgewachsen werden. Alternativ ist es möglich, die Schicht als separate Schicht auszubilden, wobei zweckmäßigerweise die Unterseite des Dielektrikums strukturiert ist und die separate Schicht mit einer komplementären Strukturierung zur strukturierten Oberfläche des Dielektrikums ausgebildet ist. Die Strukturierung der Oberfläche des Dielektrikums kann aus den vorstehenden Noppen gebildet sein. Die die Matrix bildende Schicht kann aus einem vom Körper resorbierbaren Material bestehen. Ein bevorzugtes Material ist Kollagen. Die Dicke der Schicht kann dabei der Länge der vorstehenden Noppen entsprechen, da sich das Plasma auch innerhalb der Schicht ausbilden kann.

Die Erfindung soll im Folgenden anhand eines in der Zeichnung dargestellten Ausführungsbeispiels näher erläutert werden. Es zeigen:
- Figur 1: eine perspektivische Ansicht der Oberseite einer erfindungsgemäßen Elektrodenanordnung;
- Figur 2: eine perspektivische Ansicht einer Unterseite der erfindungsgemäßen Elektrodenanordnung;
- Figur 3: eine Darstellung der Elektrode innerhalb der Elektrodenanordnung mit einer gestrichelten Darstellung des Dielektrikums zur Verdeutlichung der Einbettung der Elektrode;
- Figur 4: eine Darstellung der Teile der Elektrodenanordnung mit einer Kontaktanordnung;
- Figur 5: eine perspektivische schematische Darstellung, wobei ein zungenförmiges Anschlussstück der Elektrodenanordnung noch nicht in die Kontaktanordnung eingeschoben ist;
- Figur 6: eine vergrößerte, geschnittene Darstellung der Kontaktanordnung in dem Zustand der Figur 5;
- Figur 7: die Darstellung gemäß Figur 5, jedoch mit eingeschobenem Anschlussstück der Elektrodenanordnung,
- Figur 8: eine vergrößerte, geschnittene Darstellung des Zustands gemäß Figur 7;
- Figur 9: eine Darstellung gemäß Figur 7, jedoch mit der in einer Andruckstellung des Vorspannungskontakts an einem Anschlussstück der Elektrode verriegelten Zustand der Kontaktanordnung;
- Figur 10: eine vergrößerte und geschnittene Darstellung des Zustands gemäß Figur 9.

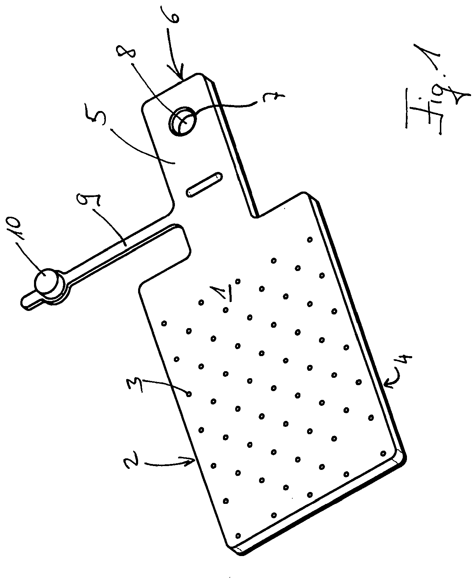

Die in Figur 1 dargestellte Elektrodenanordnung weist eine Oberseite 1 eines Dielektrikums 2 auf, über die zahlreiche Durchgangsöffnungen 3 verteilt angeordnet sind. Die Durchgangsöffnungen 3 sind in Figur 1 in einem gleichmäßigen Muster verteilt dargestellt. Dies ist für eine gleichmäßige Ableitung von Fluid von einer zu behandelnden Oberfläche vorteilhaft, jedoch nicht zwingend. Es kann für Anwendungsfälle vorteilhaft sein, eine ungleichmäßige Verteilung der Durchgangsöffnungen 3 über das Dielektrikum vorzusehen, beispielsweise im Zentrum der flächigen Elektrodenanordnung eine größere Dichte der Durchgangsöffnungen 3 als am Rand.

Das Dielektrikum 2 weist eine gegenüber der Fläche der Oberseite 1 geringe Höhe auf und die Durchgangsöffnungen 3 erstrecken sich senkrecht zur Oberseite 1 von der Oberseite 1 zu einer in Figur 1 nicht dargestellten Unterseite 4, weisen also eine Kanallänge auf, die der Höhe des Dielektrikums 2 entspricht. Die Elektrodenanordnung des dargestellten Ausführungsbeispiels besteht aus einer im Wesentlichen rechteckigen Fläche. Diese Formgebung ist jedoch nicht zwingend und kann dem jeweiligen Anwendungsfall angepasst werden. So ist es ebenso denkbar, dass die mit den Durchgangsöffnungen 3 versehene Fläche der Elektrodenanordnung oval, kreisrund oder abgewinkelt ausgebildet ist. Die Elektrodenanordnung und das Dielektrikum 2 weisen ein zungenförmiges Anschlussstück 5 auf, das als schmaler rechteckiger Steg ausgebildet ist und an einem vorderen freien Ende 6 ein zylindrische Ausnehmung 7 im Dielektrikum 2 aufweist. Am Boden der Ausnehmung 7 ist ein Teil eines leitenden Anschlussstücks 8 freigelegt.

Mit dem zungenförmigen Anschlussstück 5 des Dielektrikums 2 ist über einen schmalen bandförmigen Ansatz 9 ein zylindrisch geformter Stopfen 10 einstückig mit dem Dielektrikum 2 verbunden. Der Stopfen 10 dient zum Verschließen der zylindrischen Ausnehmung 7, um die leitende Oberfläche des leitenden Anschlussstücks 8 vor Verschmutzung zu bewahren.

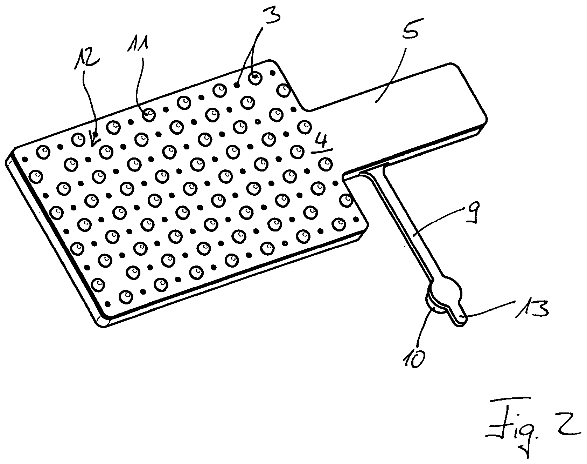

Figur 2 zeigt die entsprechende Unterseite 4 des Dielektrikums 2. Auf der Unterseite 4 sind die freien Mündungsöffnungen der Durchgangsöffnungen 3 erkennbar. Weiterhin ist die Unterseite 4 mit einer Vielzahl vorstehender Noppen 11 versehen, die zwischen sich einen zusammenhängenden Freiraum 12 ausbilden, wenn die Noppen auf eine zu behandelnde Oberfläche aufliegen. Die Noppen 11 bewirken dabei, dass sich im Freiraum ausreichend Gas bzw. Luft befindet, in dem sich das angestrebte Plasma ausbilden kann.

Figur 2 verdeutlicht noch, dass der Stopfen 10 mit einer mit dem bandförmigen Ansatz 9 fluchtenden überstehenden Lasche 13 versehen ist, an der er aus der zylindrischen Ausnehmung 7 herausziehbar ist, wenn die Elektrodenanordnung in Betrieb genommen werden soll.

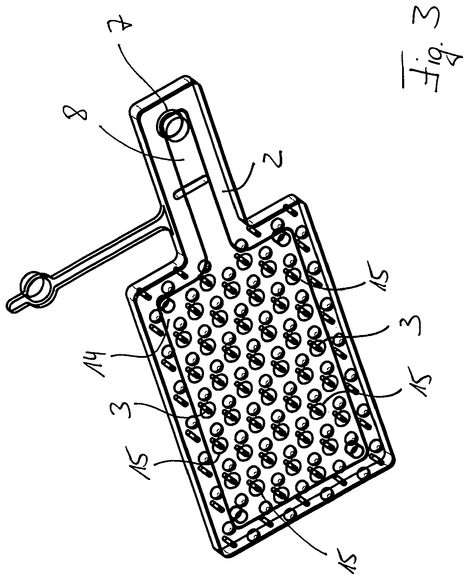

Figur 3 verdeutlicht, dass in dem Dielektrikum 2 eine flache, flexible Elektrode 14 eingebettet ist, die im Wesentlichen die Form des Dielektrikums 2, jedoch allseitig mit kleineren Abmessungen aufweist, sodass das Dielektrikum 2 die Elektrode 14 allseitig isolierend umgibt. Die Elektrode 14 besteht aus einem flächigen, flexiblen leitenden Material. Die Flexibilität kann sich aus einer elastischen Verformbarkeit, aber auch aus einer plastischen Verformbarkeit des Materials der Elektrode 14 ergeben. Die Elektrode 14 ist in den im Wesentlichen rechteckig ausgebildeten Bereich mit Durchgangsöffnungen 15 versehen, die mit den Durchgangsöffnungen 3 des Dielektrikums 2 fluchten, jedoch wesentlich größere Abmessungen aufweisen. Das Dielektrikum 2 ist so ausgebildet, dass es sich durch die Durchgangsöffnungen 15 hindurch erstreckt und somit in den Durchgangsöffnungen 15 rohrförmige Kanäle bildet, die ausschließlich von einer ausreichenden Wandstärke des Dielektrikums 2 begrenzt sind. Auf diese Weise kann durch die Durchgangsöffnungen 3 des Dielektrikums 2 ein Fluid hindurchtreten, ohne in einen direkten Kontakt oder in eine zu große Nähe zu dem Material der Elektrode 14 zu geraten.

Die Elektrode 14 ist einstückig mit dem Anschlussstück 8 verbunden, das sich ebenfalls zungenförmig in das zungenförmige Anschlussstück 5 soweit erstreckt, dass es den Boden der zylindrischen Ausnehmung 7 bilden kann. Auf diese Weise ist die Elektrode durch die zylindrische Ausnehmung 7 hindurch kontaktierbar.

Das Dielektrikum 2 kann die Elektrode 14 einstückig umschließen, indem die Elektrode in einem Spritzvorgang allseitig mit dem Dielektrikum umspritzt oder umgossen wird.

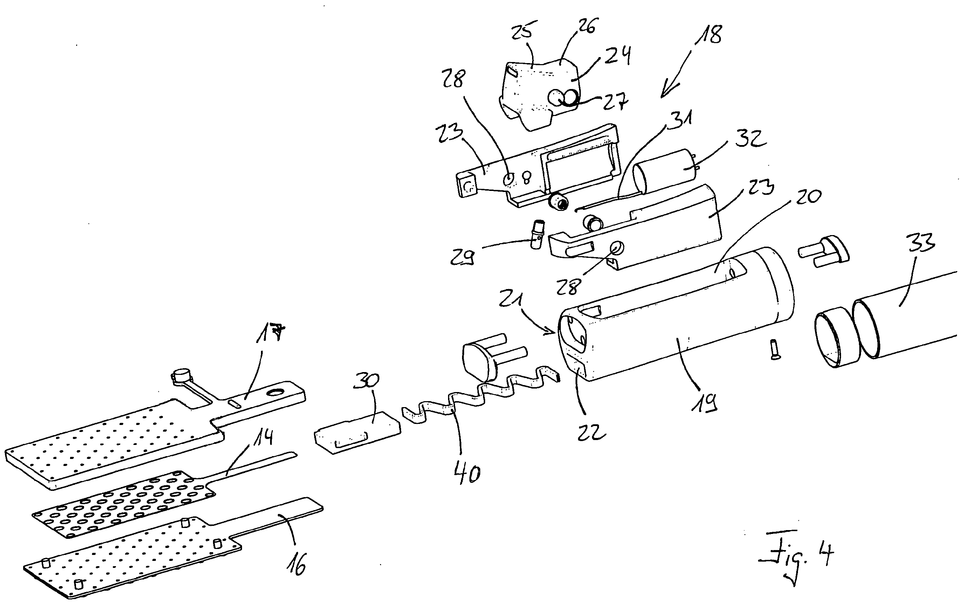

Figur 4 verdeutlicht jedoch, dass es für eine Vereinfachung des Spritzgusswerkzeugs vorteilhaft sein kann, wenn das Dielektrikum aus einem Oberteil 16 und einem Unterteil 17 hergestellt ist. Die Elektrode 14 kann dabei in das wannenförmig ausgebildete Oberteil 16 eingelegt werden.

Anschließend wird das Unterteil 17 aufgelegt und vorzugsweise materialschlüssig mit dem Oberteil verbunden, sei es durch Verklebung oder Verschweißung. Es ist auch möglich, das Oberteil 16 mit der eingelegten Elektrode in ein Spritzgusswerkzeug für das Unterteil 17 einzulegen und dann das Unterteil auszuformen, wobei es mit dem identischen Material des Oberteils 16 ein einheitliches Bauteil bildet. Selbstverständlich kann auch umgekehrt verfahren und die Elektrode zunächst in ein wannenförmiges oder geeignet geformtes Unterteil 17 eingelegt werden, bevor das Oberteil 16 aufgelegt und mit dem Unterteil 17 verbunden wird.

Die Zuführung von Hochspannung zu der Elektrode 14 über die den Boden der zylindrischen Ausnehmung 7 bildende Oberfläche des Anschlussstücks 8 der Elektrode 14 erfolgt mit einer Kontaktanordnung 18, deren Einzelteile in Figur 4 dargestellt sind. Die Kontaktanordnung 18 weist ein rohrförmiges Gehäuse 19 auf, das eine nach oben offene Kammer 20 ausbildet. Ein freies vorderes Stirnende 21 des Gehäuses ist mit einem Einführungsschlitz 22 versehen, durch den das zungenförmige Anschlussstück 5 des Dielektrikums 2 in die Kammer 20 einführbar ist. In die Kammer 20 sind zwei Seitenstücke 23 einsetzbar, zwischen denen ein Kippschalter 24 gelagert wird. Der Kippschalter 24 weist in bekannter Weise an seiner Oberseite zwei im Winkel zueinander stehende Betätigungsflächen 25, 26 auf. Aus den Seitenwänden des Kippschalters ragen zwei zylindrische Achsstummel 27 heraus, die in entsprechenden kreisförmigen Ausnehmungen 28 einsetzbar sind und die drehbare Bewegung des Kippschalters 24 ermöglichen. Mit dem Kippschalter ist ein zylindrischer Hochspannungskontakt 29 verbunden, der vom Kippschalter nach unten ragt und durch eine verschiebbare Isolierplatte 30 nach unten abdeckbar ist. Die verschiebbare Isolierplatte 30 ist in entsprechenden (nicht dargestellten) Führungen der Seitenstücke 23 geführt und durch eine Druckfeder 40 in Fig. 4 nach links mit einer Kraft beaufschlagt.

Der Hochspannungskontakt 29 ist in dem Kippschalter 24 mit einer Ausgangsleitung 31 eines Hochspannungstransformators 32 verbunden, der aus einer ihm zugeführten Netz-Wechselspannung eine Hochspannungs-Wechsel-spannung transformiert.

Die hintere Stirnseite des Gehäuses 19 ist offen und mit einem Schutzkabelmantel 33 verbunden, durch das eine Netzleitung mit dem Hochspannungstransformator 32, der in der Kammer 20 am hinteren Ende angeordnet ist, verbindbar ist.

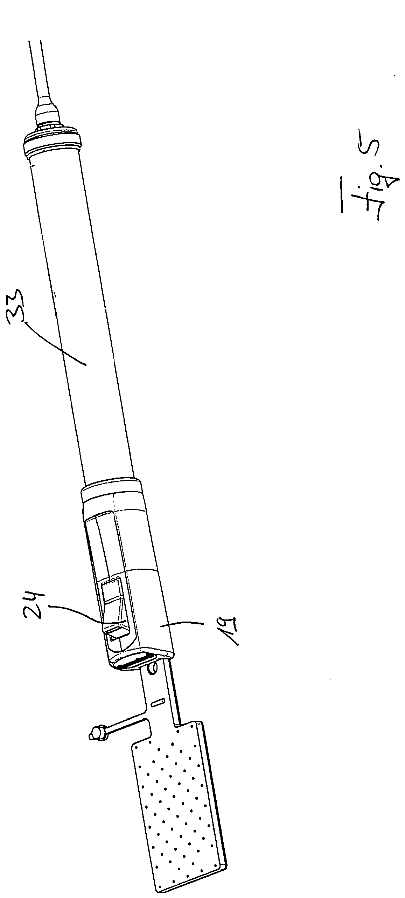

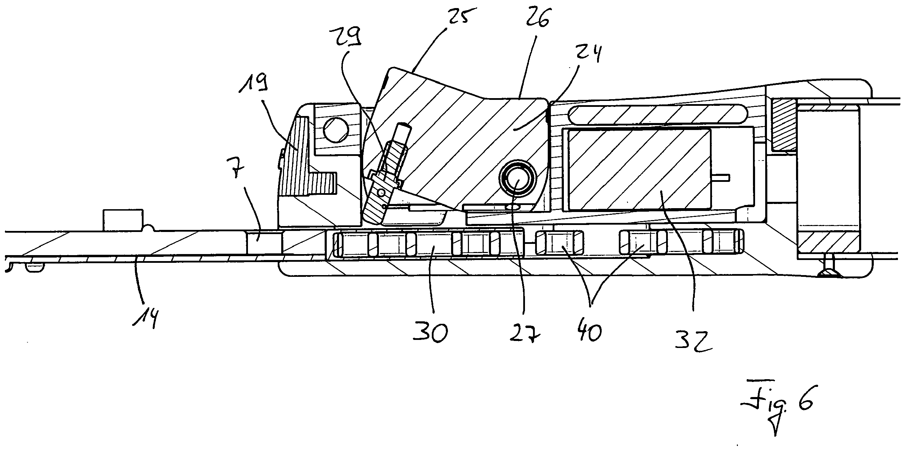

Figur 5 und 6 verdeutlichen einen Ausgangszustand der Kontaktanordnung, in der sich die verschiebbare Isolierplatte 30 am freien Ende des Gehäuses 19 unmittelbar hinter dem Einführungsschlitz 22 befindet, sodass sich Ansatzstücke 34 des Kippschalters 24 auf der Isolierplatte abstützen und eine Betätigung des Kippschalters 24 an der vorderen Betätigungsfläche 25 verhindert. In diesem Zustand liegt auch der zylindrische Hochspannungskontakt 29 auf der verschiebbaren Isolierplatte 30 auf.

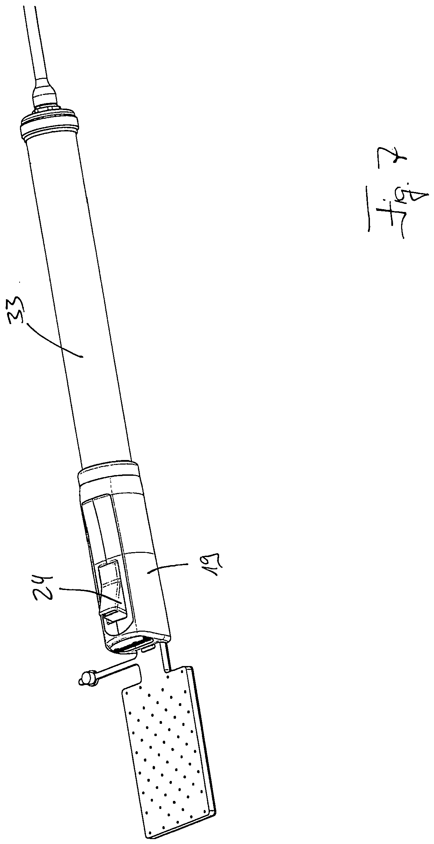

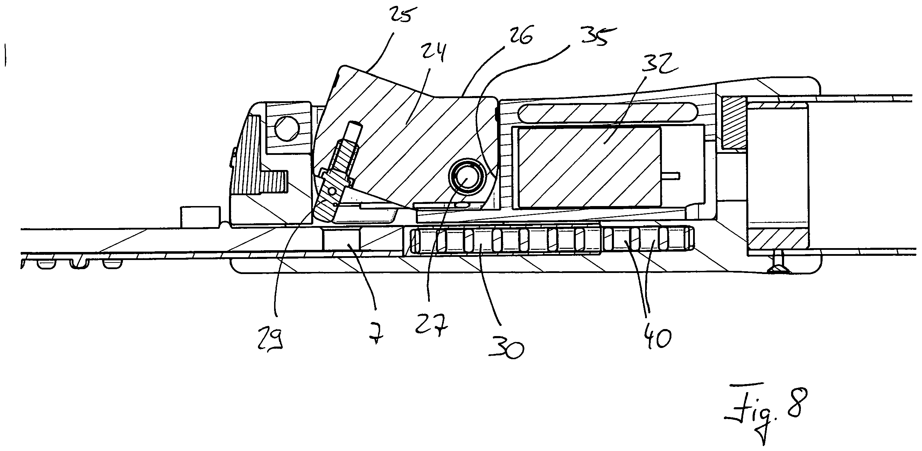

Die Figuren 7 und 8 verdeutlichen einen Zwischenzustand, in dem das zungenförmige Anschlussstück 5 des Dielektrikums 2 in den Einführungsschlitz 22 des Gehäuses 19 der Kontaktanordnung 18 eingeschoben ist. Dadurch ist die verschiebbare Isolierplatte 30 nunmehr nach hinten verschoben und hat den Hochspannungskontakt 29 freigegeben, der nun auf der Oberseite 1 des Anschlussstücks 5 des Dielektrikums 2 gleitet, bis er über der zylindrischen Ausnehmung 7 zum Liegen kommt. In diesem Zustand kann der Kippschalter 24 durch Drücken auf die vordere Betätigungsfläche 25 an seinem vorderen Ende nach unten gedrückt werden, sodass der Hochspannungskontakt 29 in die zylindrische Ausnehmung 7 einfährt und mit seinem vorderen Stirnende gegen die Oberseite des Anschlussstücks 8 der Elektrode 14 zur Anlage kommt. Figur 8 verdeutlicht, dass der Kippschalter 24 eine hintere, nockenartig geformte Abschlusswand 35 aufweist, die zusammen mit einer senkrecht stehenden Gehäusezwischenwand 36 eine Bewegung des Kippschalters 24 über einen Widerstand hinweg in eine verriegelte Klemmstellung ermöglicht, wenn der Kippschalter 24 über die fordere Betätigungsfläche 25 nach unten gedrückt wird und um die Achsstummel 27 dreht.

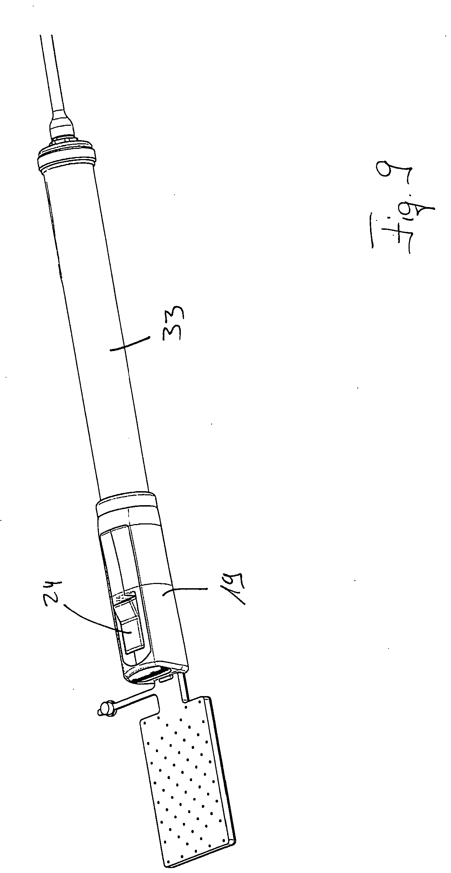

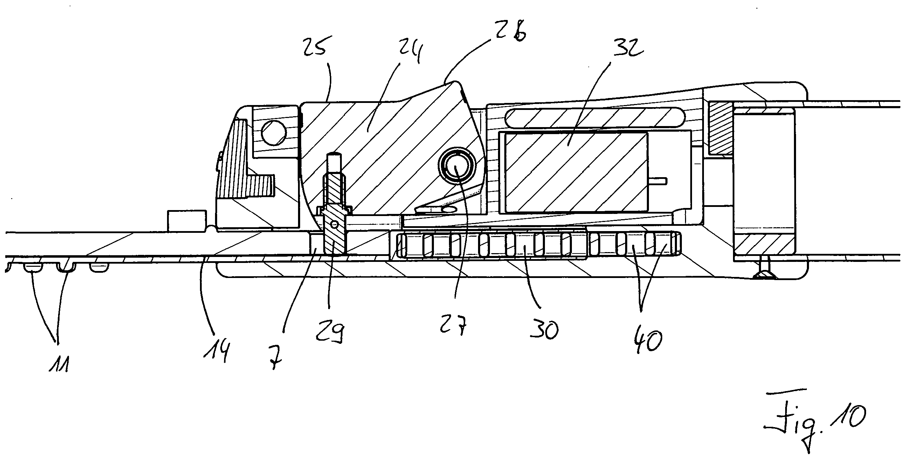

Die Figuren 9 und 10 verdeutlichen einen Klemmzustand der Kontaktanordnung 18, in dem die vordere Betätigungsfläche 25 nunmehr horizontal liegt und die hintere Betätigungsfläche etwas nach oben steht. In dem in Figuren 9 und 10 dargestellten Klemmzustand der Kontaktanordnung 18 kontaktiert der Hochspannungskontakt 29 die Elektrode 14 an deren zungenförmigen Anschlussstück 8, sodass die Elektrode 14 in diesem Zustand mit der vom Hochspannungstransformator 32 generierten Hochspannung versorgt wird.

In dem dargestellten Ausführungsformbeispiel wird die Hochspannung durch den Hochspannungstransformator 32 im Gehäuse 19 der Kontaktierungsanordnung 18 generiert, um die Wege für die Hochspannungsleitung kurz und sicher isoliert zu halten. Selbstverständlich ist es auch möglich, die Hochspannung außerhalb des Gehäuses 19 zu generieren und über ein ausreichend isoliertes und geschütztes Zuleitungskabel in das Gehäuse zum Hochspannungskontakt 29 zu leiten.

Die vorliegende Erfindung ist ferner nicht auf das dargestellte Ausführungsbeispiel beschränkt. Insbesondere ist es für die Behandlung bestimmter Oberflächen nicht erforderlich, eine Unterseite 4 des Dielektrikums 2 mit Noppen 11 zu versehen.

Die Anzahl der Durchgangsöffnungen 3 in dem Dielektrikum 2, über die sowohl Gase als auch Flüssigkeiten abgeführt und ggf. Frischluft oder Behandlungsgase in einen Wundbereich eingeleitet werden können, kann nach Anwendungsfall stark variiert werden. Die Anzahl kann daher ohne weiteres zwischen 2 und 100 schwanken, je nach Größe der Elektrodenanordnung und nach dem jeweiligen Anwendungsfall. Für den Fachmann ist es erkennbar, dass für größere Elektrodenanordnungen auch eine darüber hinausgehende Anzahl von Durchgangsöffnungen 3 realisierbar und ggf. angezeigt ist.

Die dargestellte Kontaktanordnung 18 ist für die Hochspannungskontaktierung der Elektrode vorteilhaft, kann jedoch zahlreichen konstruktiven Variationen unterworfen werden. Ferner ist es durchaus möglich, die Elektrode 14 auch mit einem Anschlussstück 5 zu versehen, das vollständig aus dem Dielektrikum 2 herausragt. Alternativ ist es darüber hinaus möglich, das Dielektrikum ohne eine Ausnehmung 7 auszubilden und stattdessen einen oder mehrere Hochspannungskontakte 29 schneidend auszubilden, sodass die Hochspannungskontaktierung sich durch das Dielektrikum 2 schneidet, bis es auf der Oberseite der Elektrode 14 zum Liegen kommt. Weitere konstruktive Varianten sind im Rahmen der Erfindung realisierbar.

Die erfindungsgemäße Elektrodenanordnung eignet sich zur unmittelbaren Auflage auf der menschlichen Haut und insbesondere auf flächigen Wunden. Für diesen Anwendungsfall ist es besonders vorteilhaft, das Dielektrikum auf der Unterseite mit einer Schicht aus einem hautfreundlichen und hautpflegenden Material zu versehen. Diese Schicht kann auf dem Dielektrikum zum Aufwachsen gebracht werden, sodass die Schicht mit dem Dielektrikum 2 verbunden ist. Die Schicht kann aber auch als separates Teil hergestellt und mit der Unterseite 4 des Dielektrikums 2 verbunden werden. Infrage kommen dabei alle Arten von Wundauflagen. Bevorzugt ist dabei eine Schicht aus einer festen, offenporigen Matrix aus einem pflegenden oder heilungsfördernden Material, beispielsweise aus Kollagen.

## Patentansprüche

1. Elektrodenanordnung zur Ausbildung einer dielektrisch behinderten Plasmaentladung zwischen einer flächigen Oberfläche (4) der Elektrodenanordnung und einer als Gegenelektrode dienenden, zu behandelnden Oberfläche, an der sich ein Fluid ansammeln kann, mit einer flächigen Elektrode (14), die mittels eines Anschlusses mit einer Hochspannungsquelle verbindbar ist und die in einem flächigen Dielektrikum (2) mit Ausnahme des Anschlusses für die Hochspannungsquelle vollständig eingebettet ist, wobei das flächige Dielektrikum (2) eine Oberseite (1) und eine Unterseite (4) ausbildet, die zur zu behandelnden Oberfläche als flächige Oberfläche zeigt,
und das flächige Dielektrikum (2) mit sich von der Unterseite zur Oberseite erstreckenden Durchgangsöffnungen (3) versehen ist,
wobei die flächige Elektrode (14) aus einem flächigen, flexiblen, leitenden Material besteht, **dadurch gekennzeichnet, dass** in der Fläche der flächigen Elektrode (14) verteilte, zur Ableitung von Fluid von der zu behandelnden Oberfläche ausgebildete Durchgangsöffnungen (15) im Material der flächigen Elektrode vorgesehen sind, und dass die Durchgangsöffnungen (3) des flächigen Dielektrikums (2) mit den Durchgangsöffnungen (15) der flächigen Elektrode (14) fluchten und kleinere Abmessungen als die Durchgangsöffnungen (15) der flächigen Elektrode (14) aufweisen, sodass das flächige Dielektrikum (2) auch im Bereich der Durchgangsöffnungen (15) der flächigen Elektrode (14) die flächige Elektrode (14) vollständig abdeckt.

2. Elektrodenanordnung nach Anspruch 1, wobei die die Unterseite (4) des flächigen Dielektrikums (2) mit vorstehenden Noppen (11) versehen ist, die die Höhe eines Freiraums (12) definieren, wenn die Noppen (11) auf der zu behandelnden Oberfläche aufliegen.

3. Elektrodenanordnung nach Anspruch 2, wobei sich die Durchgangsöffnungen (3) des flächigen Dielektrikums (2) zwischen den Noppen (11) befinden.

4. Elektrodenanordnung nach einem der Ansprüche 1 bis wobei das flächige Dielektrikum (2) aus einem gießbaren Kunststoff besteht.

5. Elektrodenanordnung nach einem der Ansprüche 1 bis 4, die mit einer Kontaktanordnung (18) verbunden ist, wobei der von dem flächigen Dielektrikum freiliegende Anschluss der flächigen Elektrode (14) mit der Kontaktanordnung (18) verbunden ist, die den Anschluss klemmend und isolierend übergreift.

6. Elektrodenanordnung nach Anspruch 5, wobei die Kontaktanordnung (18) einen klemmenden Zustand und einen Ausgangszustand aufweist, wobei im klemmenden Zustand ein Hochspannungskontakt (29) mit einer Vorspannung gegen den Anschluss der flächigen Elektrode (14) drückt und wobei im Ausgangszustand der Hochspannungskontakt (29) durch ein mit dem Übergang vom Ausgangszustand in den klemmenden Zustand bewegbares Isolierstück (30) abgedeckt ist.

7. Elektrodenanordnung nach Anspruch 5 oder 6, wobei die Kontaktanordnung (18) ein Gehäuse (19) mit einem einseitig offenen Schlitz (22) aufweist und dass in dem Gehäuse (19) eine Klemmanordnung (24) zum Andrücken des Hochspannungskontakts (29) an den Anschuss der flächigen Elektrode (14) gelagert ist.

8. Elektrodenanordnung nach Anspruch 6 oder 7, wobei der Hochspannungskontakt (29) ein zylindrischer Kontakt ist, der in eine entsprechende Ausnehmung (7) des flächigen Dielektrikums (2) im klemmenden Zustand eingreift und wobei die Ausnehmung (7) bis zu einem flächigen Anschlussstück (8) der flächigen Elektrode (14) ragt.

9. Elektrodenanordnung nach einem der Ansprüche 1 bis 8, wobei die Unterseite (4) des flächigen Dielektrikums (2) mit einer Wundauflage beschichtet ist.

10. Elektrodenanordnung nach Anspruch 9, wobei die Wundauflage durch eine Schicht aus einer festen, offenporigen Matrix aus einem pflegenden oder heilungsfördernden Material besteht.

## Claims

1. An electrode arrangement for forming a dielectric barrier plasma discharge between a flat surface (4) of the electrode arrangement and a surface to be treated which functions as a counterelectrode and on which a fluid can collect, comprising a flat electrode (14) which can be connected to a high voltage source by means of a connector and which is completely embedded in a flat dielectric (2), except for the connector for the high voltage source, wherein the dielectric (2) forms a top surface (1) and a bottom surface (4) being a flat surface, facing the surface to be treated, **characterized in that** the flat electrode (14) has through-holes (15) distributed over its plane, and the dielectric (2) is provided with through-holes (3) which extend from the bottom surface (4) to the top surface (1), align with the through-holes (15) of the electrode (14), and have smaller dimensions than the through-holes (15) of the electrode (14), whereby the dielectric (2) completely covers the electrode (14) also in the region of the through-holes (15).

2. The electrode arrangement as claimed in claim 1, **characterized in that** the bottom surface (4) of the dielectric (2) is provided with protuberances (11) which define the height of an open space (12) when the protuberances (11) rest on the surface to be treated.

3. The electrode arrangement as claimed in claim 1 or 2, **characterized in that** the through-holes (3) are located between the protuberances (11).

4. The electrode arrangement as claimed in one of claims 1 to 3, **characterized in that** the dielectric (2) consists of a castable plastic.

5. The electrode arrangement as claimed in one of claims 1 to 4, which is connected to a contact arrangement (18), **characterized in that** the connector of the electrode (14) free from the dielectric is connected to the contact arrangement (18) which engages over the connector in a clamping and insulating manner.

6. The electrode arrangement as claimed in claim 5, **characterized in that** the contact arrangement (18) has a clamping state and an initial state, a high voltage contact (29) presses with a preload against the connector of the electrode (14) in the clamping state and, in the initial state, the high voltage contact (29) is covered by an insulating piece (30) which is movable with the transition from the initial state into the clamping state.

7. The electrode arrangement as claimed in claim 5 or 6, **characterized in that** the contact arrangement (18) comprises a housing (19) having a slot (22) which is open on one side, and a clamping arrangement (24) for pressing the high voltage contact (29) against the connector of the electrode (14) is mounted in the housing (19).

8. The electrode arrangement as claimed in claim 6 or 7, **characterized in that** the high voltage contact (29) is a cylindrical contact which engages into a corresponding recess (7) of the dielectric (2) in the clamping state, and the recess (7) extends up to a flat connector piece (8) of the electrode (14).

9. The electrode arrangement as claimed in one of claims 1 to 8, **characterized in that** the bottom side (4) of the dielectric (2) is coated with a wound dressing.

10. The electrode arrangement as claimed in claim 9, **characterized in that** the wound dressing consists of a layer made from a solid, open-pore matrix made from a therapeutic or curative material.

## Revendications

1. Ensemble d'électrode pour engendrer une décharge de plasma à barrière diélectrique entre une surface plate (4) de l'ensemble d'électrode et une surface à traiter qui sert de contre-électrode et sur laquelle un fluide peut s'accumuler, comportant une électrode plate (14) qui peut être connectée à une source de haute tension au moyen d'une borne de connexion et qui est entièrement noyée dans un diélectrique plat (2), à l'exception de la borne de connexion pour la source de haute tension, dans lequel
le diélectrique plat (2) forme une face supérieure (1) et une face inférieure (4) dirigée vers la surface à traiter en tant que surface plate, et
le diélectrique plat (2) est pourvu d'ouvertures traversantes (3) s'étendant de la face inférieure vers la face supérieure,
l'électrode plate (14) est constituée d'un matériau plat, flexible, conducteur, **caractérisé en ce que**
des ouvertures traversantes (15) réparties sur sa surface de l'électrode plate (14), ménagées pour évacuer du fluide depuis la surface à traiter, sont prévues dans le matériau de l'électrode plate, et **en ce que** les ouvertures traversantes (3) du diélectrique plat (2) sont en alignement avec les ouvertures traversantes (15) de l'électrode plate (14) et présentent des dimensions inférieures à celles des ouvertures traversantes (15) de l'électrode plate (14), de sorte que le diélectrique plat (2) recouvre complètement l'électrode plate (14) également dans la zone des ouvertures traversantes (15) de l'électrode plate (14).

2. Ensemble d'électrode selon la revendication 1,
dans lequel la face inférieure (4) du diélectrique plat (2) est munie de boutons saillants (11) qui définissent la hauteur d'un espace libre (12) lorsque les boutons (11) reposent sur la surface à traiter.

3. Ensemble d'électrode selon la revendication 2,
dans lequel les ouvertures traversantes (3) du diélectrique plat (2) sont situées entre les boutons (11).

4. Ensemble d'électrode selon l'une des revendications 1 à 3,
dans lequel le diélectrique plat (2) est constitué d'une matière plastique coulable.

5. Ensemble d'électrode selon l'une des revendications 1 à 4,
qui est relié à un ensemble de contact (18), la borne de connexion de l'électrode plate (14), mise à découvert du diélectrique plat, étant reliée à l'ensemble de contact (18) qui coiffe la borne de connexion en la serrant et en l'isolant.

6. Ensemble d'électrode selon la revendication 5,
dans lequel l'ensemble de contact (18) présente un état de serrage et un état initial, et, dans l'état de serrage, un contact haute tension (29) s'appuie sous précontrainte contre la borne de connexion de l'électrode plate (14), et dans l'état initial, le contact haute tension (29) est recouvert par une pièce isolante (30) qui peut être déplacée lors du passage de l'état initial à l'état de serrage.

7. Ensemble d'électrode selon la revendication 5 ou 6,
dans lequel l'ensemble de contact (18) présente un boîtier (19) ayant une fente (22) ouverte sur un côté, et
un ensemble de serrage (24) pour presser le contact haute tension (29) contre la borne de connexion de l'électrode plate (14) est monté dans le boîtier (19).

8. Ensemble d'électrode selon la revendication 6 ou 7,
dans lequel le contact haute tension (29) est un contact cylindrique qui, à l'état de serrage, s'engage dans un évidement correspondant (7) du diélectrique plat (2), et l'évidement (7) s'étend jusqu'à une pièce de connexion plate (8) de l'électrode plate (14).

9. Ensemble d'électrode selon l'une des revendications 1 à 8,
dans lequel la face inférieure (4) du diélectrique plat (2) est revêtue d'un pansement.

10. Ensemble d'électrode selon la revendication 9,
dans lequel le pansement est constitué d'une couche d'une matrice solide à pores ouverts en un matériau de soin ou de cicatrisation.
